# EUROPEAN PATENT SPECIFICATION

(11) **EP 1 547 634 B1**
(45) Date of publication and mention of the grant of the patent: **15.07.2009**
(21) Application number: 04105886.8
(22) Date of filing: 18.11.2004
(51) Int. Cl.: A61M 5/32, A61M 5/50

(54) **Safety syringe**
Sicherheitsspritze
Seringue de sécurité

(30) Priority: 22.12.2003 CN 200310123297
(43) Date of publication of application: 29.06.2005
(73) Proprietor: Wang, Chih Ming, Keelung City (TW); Tseng, Hsi-Hsun, Keelung City (TW); Lee, Po-Liang, Keelung City (TW); Chuang, Chun-Chieh, Keelung City (TW)
(72) Inventor: Yang, Chung-Yu, Taipei City (TW)
(74) Representative: 2K Patentanwälte Blasberg Kewitz & Reichel

(56) References cited:
- WO-A-01/49348
- WO-A-20/04105842
- US-A1- 2003 163 094
- US-A1- 2003 212 371
- US-B1- 6 221 055

## Description

### BACKGROUND OF THE INVENTION

### 1. FIELD OF THE INVENTION

The present invention relates to a medical syringe, and more particularly to a safety syringe with an automatically retractable needle.

### 2. DESCRIPTION OF THE PRIOR ART

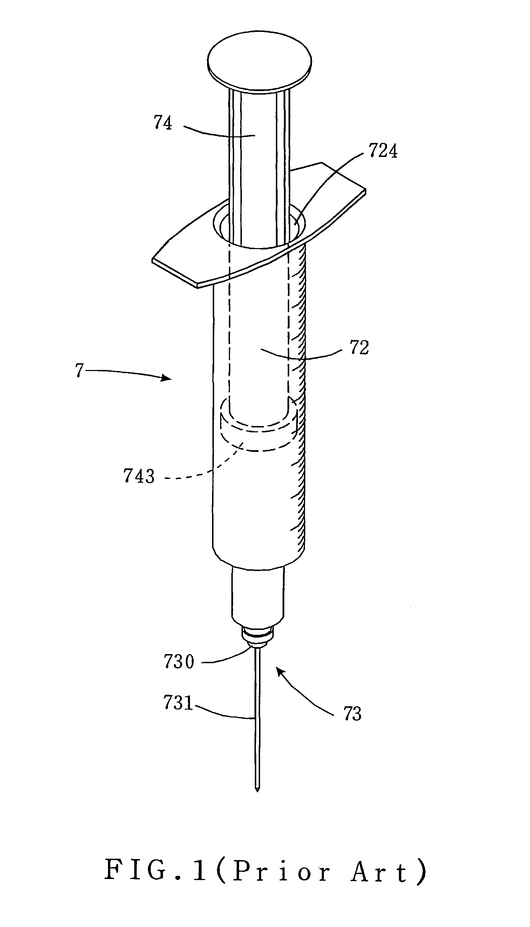

As shown in FIG. 1, a prior syringe includes a syringe barrel 72 with a hole in the front end of the syringe barrel 72 and scales on the surface of the syringe barrel 72. A needle unit 73 includes needle holder 730 with the thread of a screw for bolting the needle unit 73 in the syringe barrel 72. The rear end of the needle 731 is fastened across the needle holder 730, wherein the front end of the needle 731 is projected from the needle holder 730. The rear end of the syringe barrel 72 includes a rear opening 724 with large cross-sectional area or the diameter. A rubber-sealing ring 743 fastens a rod 74 inside the syringe barrel 72, wherein the rod 74 is movable across the rear opening 724. The external diameter of the rubber-sealing ring 743 is slightly larger than the internal diameter of the rear opening 724 and the internal diameter of the syringe barrel 72. The rubber-sealing ring 743 injects the medicament withdrawn inside the syringe barrel 72 through the needle 731 when the rod 74 is pushed.

The needle 731 may accidentally stab an operating staff while the operating staff encloses the needle 731 by a needle sheath. If the syringe 7 is used and the needle 731 comes in contact with the blood of an individual, the stabbed operating staff may become infected with diseases, e.g. AIDS, viral hepatitis b and so on.

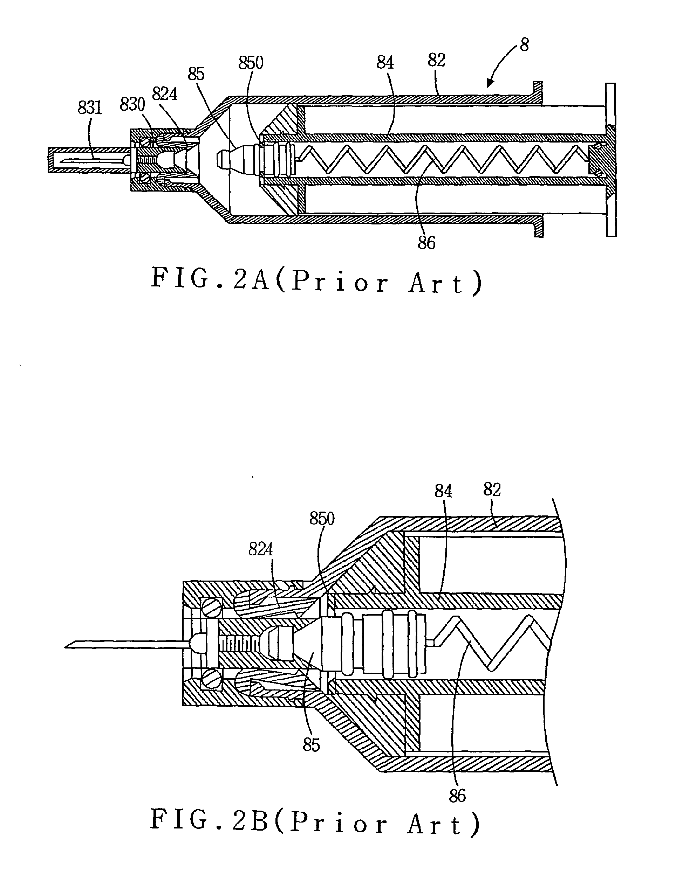

The medical appliances are improved to protect operating staff from being inflected. Safety syringes protect the operating staff from being stabbed and infected while tidying the needle 731 after injecting the medicament for patients. As shown in FIG. 2A and 2B, a safety syringe 8, which is disclosed by U.S. Patent NO. 5, 395, 337, includes a syringe barrel 82 and a spring 84. The syringe barrel 82 includes a tapered wall 824 for fastening a needle holder 830. A flexible sealing member 85 is wedged in the front end of the spring 84 by a retaining ring 850 having a lead angle. A spring 86 connects with the flexible sealing member 85 to pull the flexible sealing member 85 when the operating staff finishes the injection. As shown in FIG. 2B, the operating staff can tidy the needle 831 by pushing the spring 84 to wedge the flexible sealing member 85 by the tapered wall 824. When the tapered wall 824 wedges the flexible-sealing member 85, the flexible sealing member 85 is released from the retaining ring 850 if the operating staff continuously pushes the spring 84. The flexible sealing member 85 is pulled by the spring 86 to movably dispose inside the spring 84 after the flexible-sealing member 85 is released from the retaining ring 850.

However, the diameter of the tapered wall 824 of a syringe is very small, e.g. few millimeters, wherein the capacity of the syringe is approximately 1 cubic centimeter or 2 cubic centimeters. It is pretty difficult to push the tapered wall 824 while wedging the flexible-sealing member 85 by the tapered wall 824 without partiality. It is also difficult to set the spring 86 in the spring 84 to connect the flexible sealing member 85 with the rear end of the spring 84 of a tiny syringe 7. The complex and delicate process for fabricating elements of the tiny syringe 7 increases the cost of the tiny syringe 7. The syringe 7 may be too expensive for selling. Thus it is necessary to develop more economical safety syringes for protecting the operating staff from being stabbed.

The document US 2003/0163094 discloses a safety syringe which includes a flexible holder supporting seat that is sleeved around and that clamps a needle holder with a front end portion of a syringe barrel.

The document US 6, 221, 055 discloses a single use retractable medical device which dispenses fluids from a separable carpule.

The document WO 01/49348 A2 discloses a retracting needle syringe including a barrel having a chamber. A release element is movably connected to the flange and has a sharp proximal end projecting into the chamber. Distal motion of the plunger causes the sharp proximal end to cut through the cover element and the release element to dissociate an outer portion of the flange from an inner portion of the flange allowing the spring to expand and move the needle cannula into the cavity.

The WO 2004/105842 A1 discloses a syringe with restrictor. Located internally within the barrel, adjacent its open end, is a restrictor in the form of a resiliently deformable disc having a central opening.

The US 2003/021371A1 discloses a disposable safety syringe which includes a generally tubular body having a needle end and a plunger end, needle seat, two-way valve, plunger stopper and rear plunger seal. The plunger may be selectively engageable with both the stopper and the retractable needle seat.

### SUMMARY OF THE INVENTION

In accordance with the present invention, a safety syringe is provided to substantially overcome the drawbacks of the above mentioned problems for automatically retracting the needle unit inside a plunger by pressure as defined in claim 1.

Accordingly, it is another objective of the present invention to provide a safety syringe, which can be conveniently operated, with convenient fabricated elements for protecting the operating staff from being stabbed and infectious diseases.

### BRIEF DESCRIPTION OF THE DRAWINGS

The foregoing aspects and many of the attendant advantages of this invention will become more readily appreciated as the same becomes better understood by reference to the following detailed description, when taken in conjunction with the accompanying drawings, wherein:
FIG. 1 illustrates a schematic diagram of a safety syringe in the prior art ;
FIG. 2A illustrates a longitudinal cross-sectional view of a safety syringe incorporating the plunger in the prior ;
FIG. 2B illustrates a view similar to FIG. 2A, showing final movement of the safety syringe in the prior art ;
FIG. 3A-3G illustrate sectional views of a safety syringe of the first embodiment of the present invention ;
FIG. 4A-4E illustrate sectional views of a safety syringe of the second embodiment of the present invention ;
FIG. 5A-5D illustrate sectional views of a safety syringe of the third embodiment of the present invention ;
FIG. 6 illustrates a sectional view of a safety syringe of the fourth embodiment of the present invention ;
FIG. 7A-7D illustrate sectional views of a safety syringe of the fifth embodiment of the present invention ;
FIG. 8 illustrates a sectional view of a safety syringe of the sixth embodiment of the present invention ;
FIG. 9A-9B illustrate sectional views of a safety syringe of the seventh embodiment of the present invention ;
FIG. 10 illustrates a sectional view of a safety syringe of the eighth embodiment of the present invention ;
FIG. 11A-11C illustrate sectional views of a safety syringe of the ninth embodiment of the present invention ; and
FIG. 12 illustrates a sectional view of a safety syringe of the tenth embodiment of the present invention.

### DESCRIPTION OF THE PREFERRED EMBODIMENT

These preferred embodiments of the present invention are now described in greater detail. Nevertheless, it should be recognized that the present invention can be practiced in a wide range of other embodiments besides those explicitly described, and the scope of the present invention is expressly not limited except as specified in the accompanying claims.

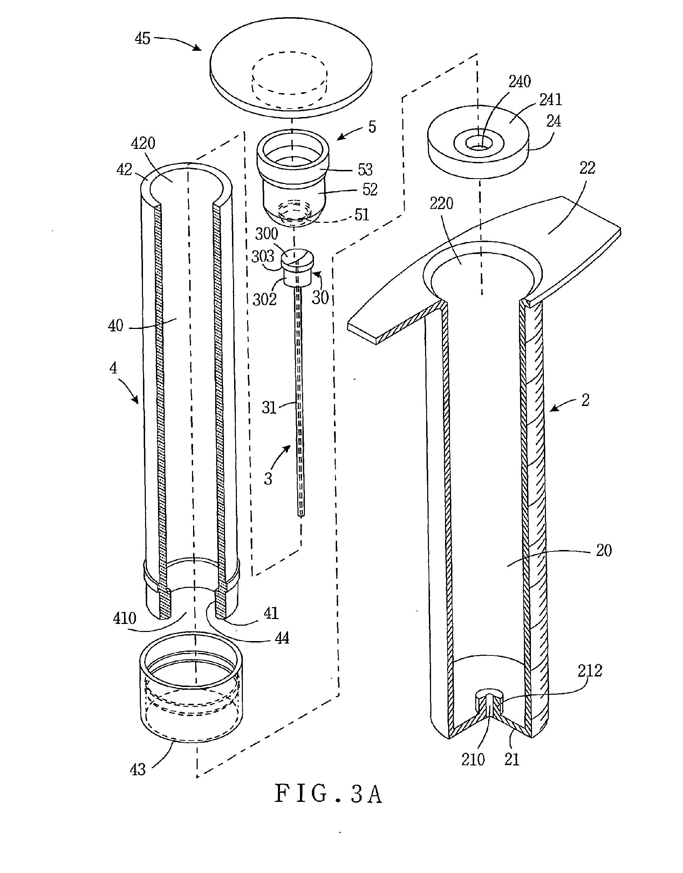

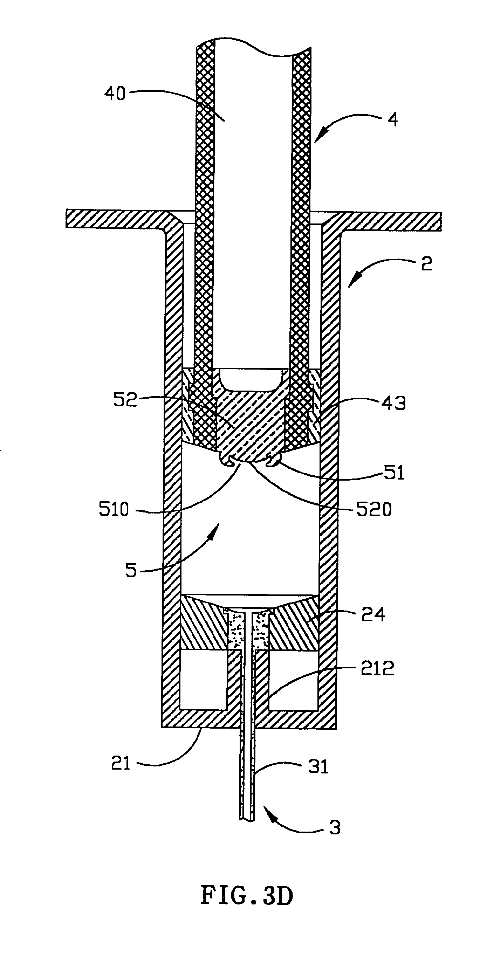

As shown in FIG. 3A-3D, the first embodiment of the present invention provides a safety syringe including a syringe barrel 2, a needle unit 3 set in the front end of the syringe barrel 2, a plunger 4 movably disposed within the syringe barrel 2 and a flexible sealing member 5 disposed movably within the front end of the plunger 4.

The syringe barrel 2 includes a barrel body 20, a front-end wall 21 and a rear end wall 22. A front opening 210 and the front-end wall 21 are integrally formed. A rear opening 220 is formed integrally with the rear end wall 22. The cross-sectional area of the rear opening 220 is slightly larger than the cross-sectional area of the plunger 4; thus the plunger 4 can be inserted into the syringe barrel 2. A spacer portion 212 is disposed on the front end wall 21, wherein the front opening 210 penetrates the spacer portion 212. The spacer portion 212 and the barrel body 20 can be formed integrally. However, the spacer portion 212 may be an independent element set on the front end of the barrel body 20. The cross-sectional area or the diameter of the front opening 210 is slightly smaller than that of the rear opening 220 and the plunger 4. The internal diameter of the syringe barrel 2 and the internal diameter of the rear opening 220 are approximately the same. A flexible holder-supporting seat 24 including a central hole 240 can be pushed into the syringe barrel 2 through the rear opening 220 to be set inside the syringe barrel 2. There is a distance between the flexible holder-supporting seat 24 and the front-end wall 21 of the barrel body 20.

A needle unit 3 includes a needle body 30 and a needle 31 set across the needle body 30. The needle body 30 is movably set inside the flexible holder-supporting seat 24. The front end of the needle 31 is set across the spacer portion 212 to pierce through the spacer portion 212.

The plunger 4 includes a plunger body 40 including an open front end 41 formed on an end of plunger body 40. The open front end 41 includes a front opening 410, wherein the internal diameter of the front opening 410 is slightly larger than the cross-sectional area or the diameter of the needle body 30. A flexible sealing member 5 is fastened inside the front opening 410 to form a sealed space inside the plunger body 40. A rear end wall 45 is fastened on another end of plunger body 40 to form the sealed space inside the plunger body 40. The plunger 4 fastened inside the barrel body 20 can be moved away from the syringe barrel 2 by pulling the rear end wall 45 to withdraw the medicament. The plunger 4 can be pushed to inject the medicament by pushing the rear end wall 45. The plunger body 40 may include a rubber seal ring 43 to fasten the syringe barrel 2 and the plunger 4 better for preventing the medicament inside syringe barrel 2 from overflowing.

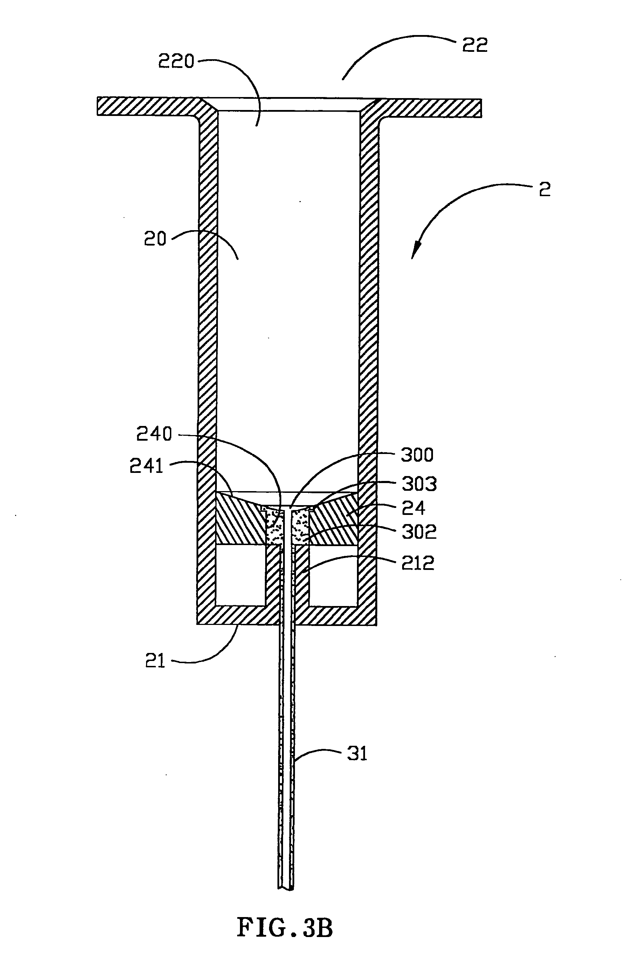

As shown in FIG. 3B, the flexible holder-supporting seat 24 is set inside the syringe barrel 2 to fasten the needle body 30 on. The needle body 30 includes a needle holder 302, a rear opening 300 and an outward flange 303, wherein the cross-sectional area or the diameter of the outward flange 303 is slightly larger than that of the needle holder 302. The cross-sectional area or the diameter of the needle holder 302 may be equal to or slightly larger than that of the spacer portion 212. The flexible holder-supporting seat 24 includes a rear end surface 241. The side of the central hole 240 of the flexible holder-supporting seat 24 fastens the needle holder 302 when the needle body 30 is stuffed into the flexible holder-supporting seat 24. The side of the central hole 240 also fastens the outward flange 303. After the needle body 30 is fastened by the flexible holder-supporting seat 24, the needle body 30 and the flexible holder-supporting seat 24 can be pushed into the syringe barrel 2 through the rear opening 220 until the needle holder 302 contacting with the spacer portion 212. The needle unit 3 set on the front end of the syringe barrel 2 is fastened well and stable because the area of the needle holder 302 contacting with the spacer portion 212 is large enough to provide enough force of stability. When the volume of the medicament withdrawn inside the syringe barrel 2 is approximately 1 cubic centimeter or 2 cubic centimeters, the layout of the prevent invention provides effective stability of the safety syringe for fastening the needle unit 3 and the syringe barrel 2.

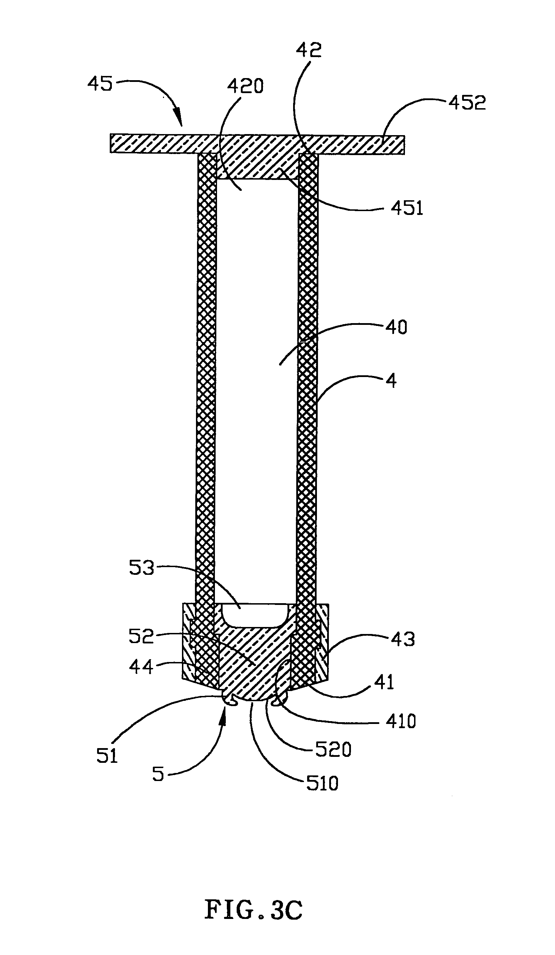

As shown in FIG. 3C, an inward flange 44 of the plunger 4 fastens the flexible sealing member 5. A rear opening 420 and the open rear end 42 are formed integrally. The plunger 4 further comprises a rear end wall 45 sealing the rear opening 420. The rear end wall 45 includes a plate 452 and a circular projection 451 extended from the plate 452. The circular projection 451 is fastened inside the plunger body 40 of the plunger 4, wherein the external diameter of the circular projection 451 is a slightly larger than the internal diameter of the rear opening 420 of the plunger 4. The flexible sealing member 5 includes a sealing rear portion 52 and a holder-retaining front portion 51 extended from the front end of the sealing rear portion 52. The flexible sealing member 5 further includes a rear end skirt portion 53 in the rear end of the flexible sealing member 5. The external diameter of the sealing rear portion 52 is slightly smaller than the internal diameter of the rear opening 420, the sealing rear portion 52 can be pushed into the rear opening 420 of the plunger body 40 thus. The external diameter of the sealing rear portion 52 is slightly larger than the internal diameter of the inward flange 44 of the plunger 4. The flexible sealing member 5 can be fastened on the open front end 41 by fastening to the inward flange 44 to seal the space inside the plunger 4. Thus, the air inside the plunger body 40 is pumped out to form a sealed space with lower pressure inside the plunger body 40, and then the rear end wall 45 is fastened on the rear opening 420 to seal the sealed space. A rubber seal ring 43 is covered on the front end of the plunger 4, wherein the external diameter of the rubber seal ring 43 is slightly larger than the internal diameter of the barrel body 20.

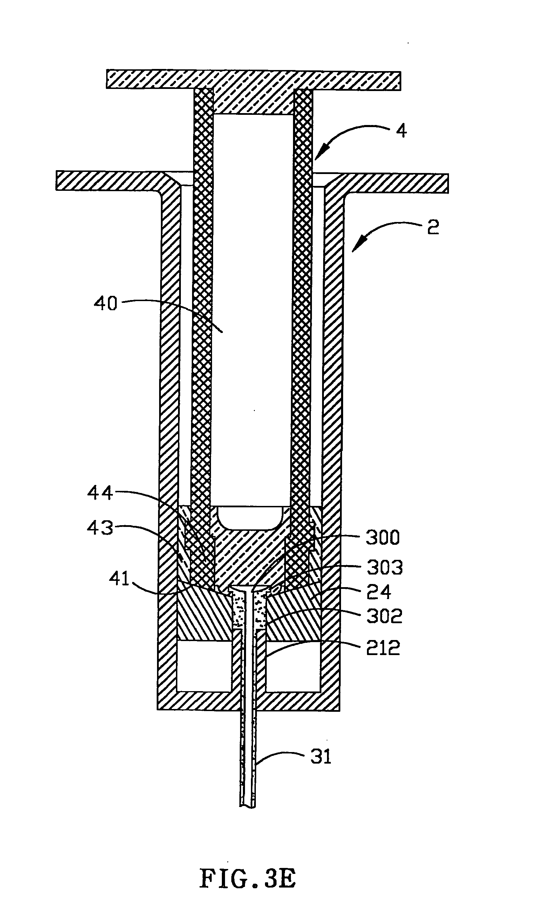

The cannular holder-retaining front portion 51 of the flexible sealing member 5 is extended forward from the sealing portion 52, and the external diameter of the holder-retaining front portion 51 is as small as the forward extension. A blind hole 510 is formed inside the holder-retaining front portion 51, wherein the bottom of the blind hold 510 is a sealing rear portion 52, and the top of the blind hole 510 is surrounded by the front end of the holder-retaining front portion 51. the internal diameter of the front end of the holder-retaining front portion 51 is slightly smaller than the external diameter of the outward flange 303. as shown in FIG. 3E, the blind hole 510 sleeves around the outward flange 303. the sealing rear portion 52 seals the rear opening 300 of the needle body 30 to stop the medicament from being injected, when the open front end 41 is pushed to contact with the flexible holder-supporting seat 24. When the open front end 41 is pushed continuously, the needle holder 302 is fastened on the spacer portion 212 but the open front end 41 continuously pushes the flexible holder-supporting seat 24. the flexible holder-supporting seat 24 does not fasten the needle body 30, if the flexible holder-supporting seat 24 is pushed continuously by the open front end 41.

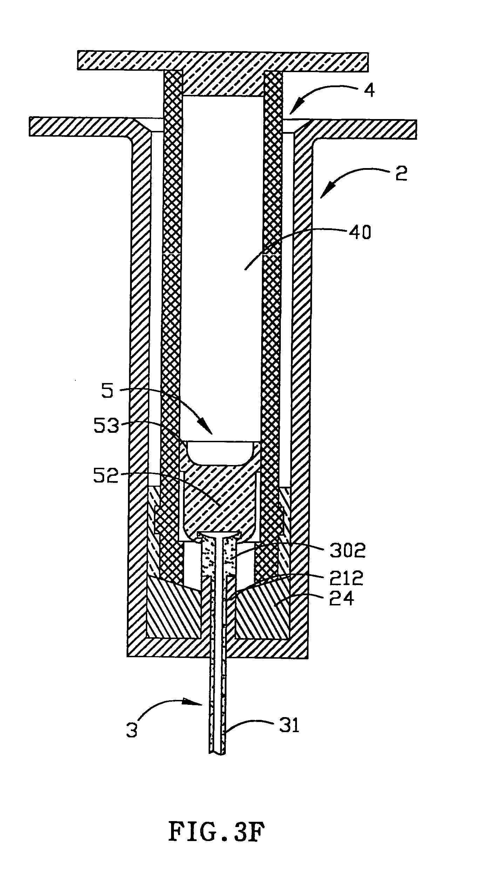

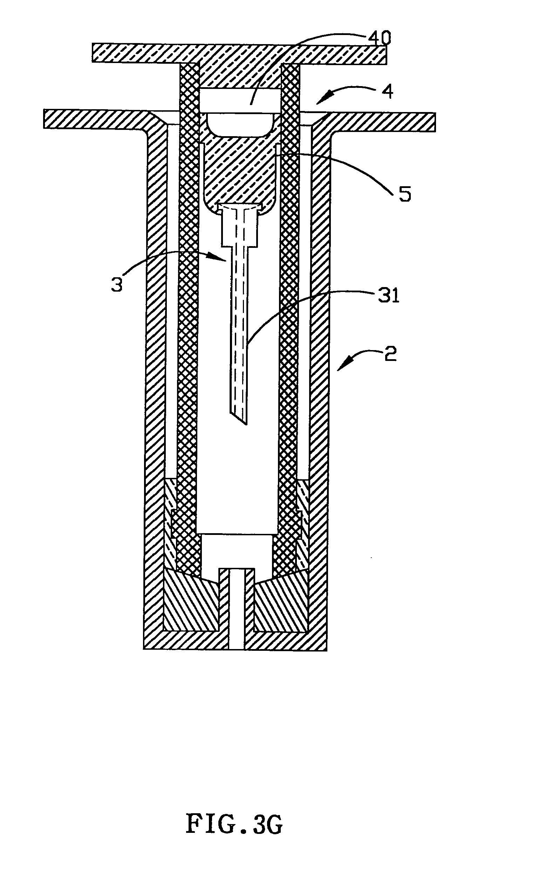

As shown in FIG. 3F, the flexible sealing member 5 stops moving because the needle body 30 props the flexible sealing member 5 up, even if the open front end 41 continuously pushes the flexible holder-supporting seat 24. The flexible sealing member 5 is released from the inward flange 44 when the inward flange 44 does not contact with the flexible sealing member 5. The pressure inside the space sealed by the sealing rear portion 52 and the plunger body 40 is lower than the pressure pulling the sealing rear portion 52. As shown in FIG. 3G, the needle unit 3 that is combined with the flexible sealing member 5 is automatically retracted into the plunger body 40 due to the different pressures. The operating staffs using the safety syringe of the present invention are protected from being stabbed. Furthermore, the sealing rear portion 52 stops air being transported into the sealed space to maintain the lower pressure of the sealed space inside the plunger body 40.

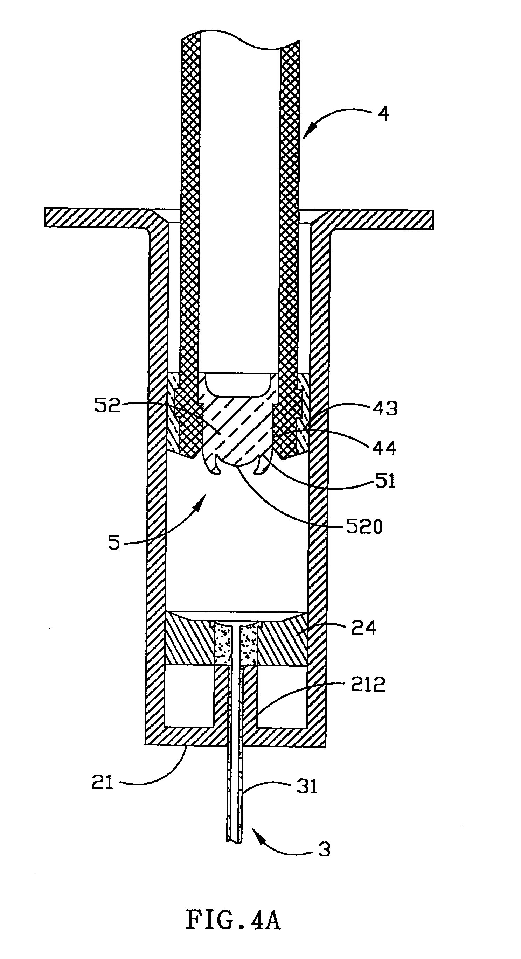

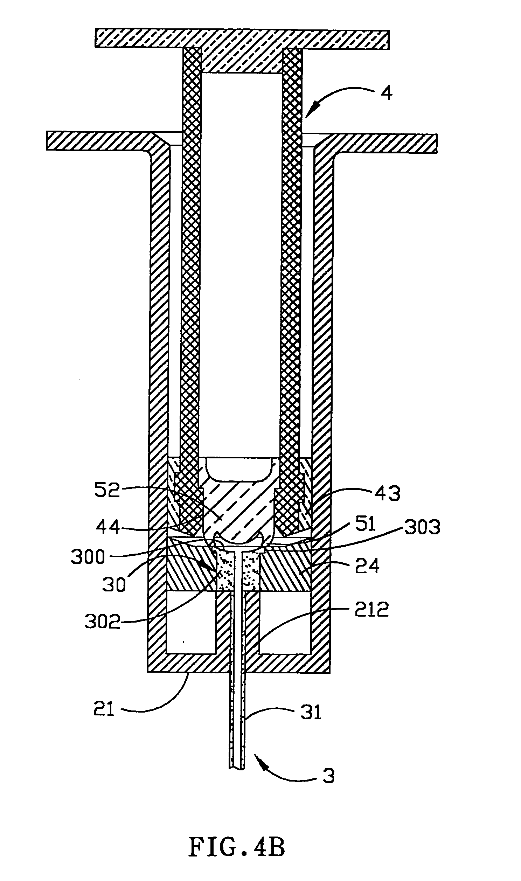

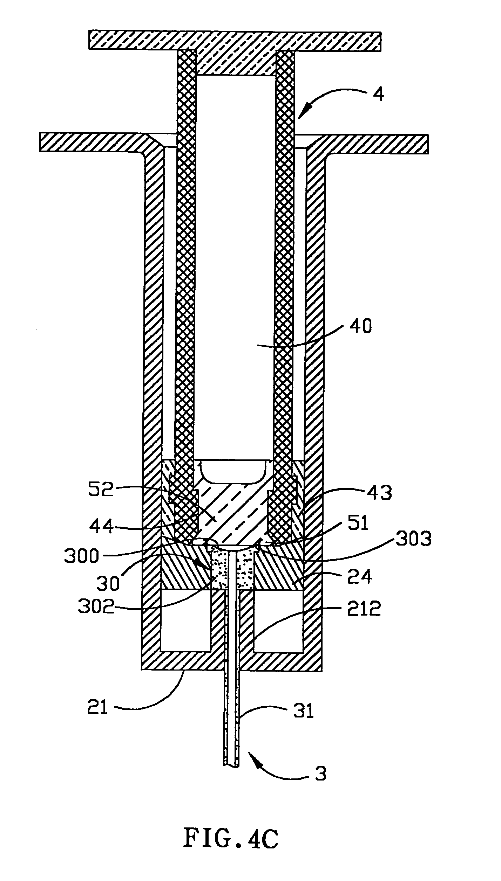

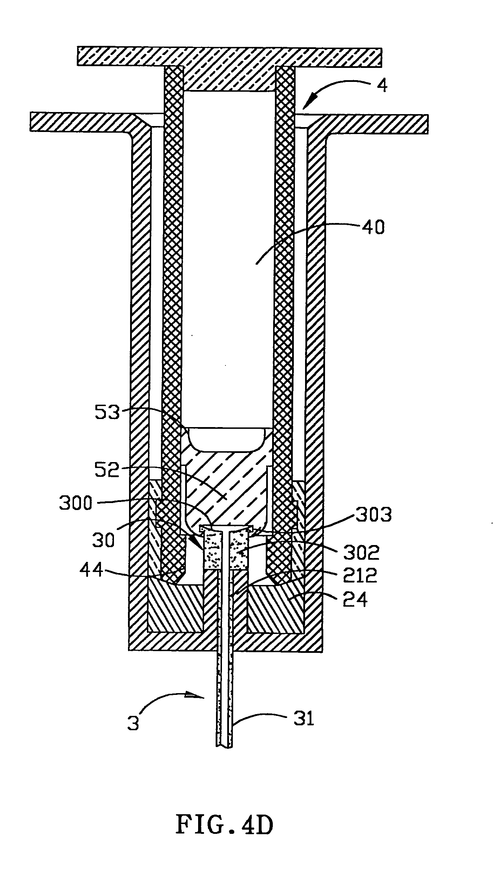

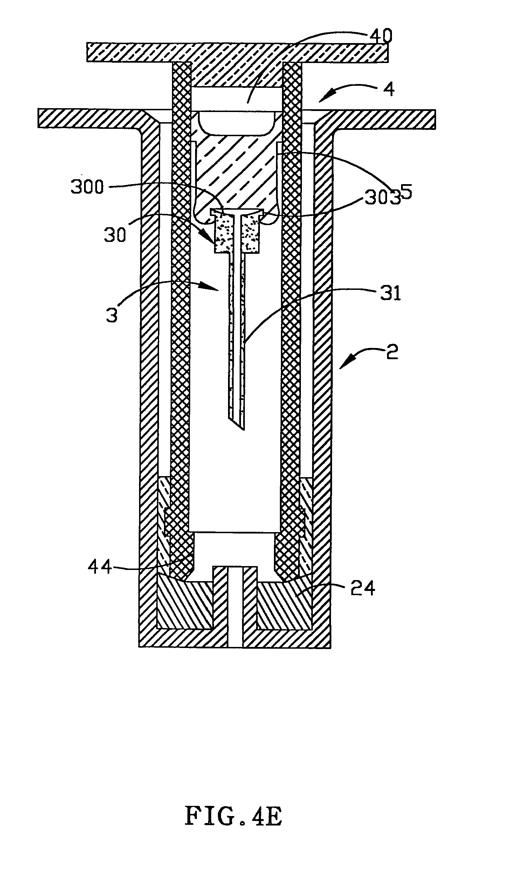

The safety syringe of the second embodiment of the present invention is illustrated as FIG. 4A-4E. The difference between the first embodiment and the second embodiment of the present invention is the length of the holder-retaining front portion 51 of the flexible-sealing member 5. The holder-retaining front portion 51 can shrink to sleeve the needle unit 3 when the plunger 4 is pushed to contact with the flexible holder-supporting seat 24. As shown in FIG. 4A, the flexible sealing member 5 includes a sealing rear portion 52 and a holder-retaining front portion 51, wherein the external diameter of the sealing rear portion 52 is slightly larger than the internal diameter of the inward flange 44. The inward flange 44 can clench the sealing rear portion 52. Thus, the side in the front end of the inward flange 44 expands slightly and does not contact with the holder-retaining front portion 51. As shown in FIG. 4B, the holder-retaining front portion 51 curves because the holder-retaining front portion 51 contacts with the flexible holder-supporting seat 24 and the needle body 30 when the plunger 4 is pushed forward to contact with the flexible holder-supporting seat 24. As shown in FIG. 4C, the curved holder-retaining front portion 51 curves continuously to be extruded in the space surrounded by the expanded inward flange 44 and the flexible holder-supporting seat 24 when the plunger 4 is continuously pushed. When the inward flange 44 is still pushed forward to push the flexible holder-supporting seat 24, the sealing rear portion 52 of the holder-retaining front portion 51 contacts with the rear opening 300 to stuff the rear opening 300, and the holder-retaining front portion 51 expands due to the outward flange 303. As shown in FIG. 4D, the holder-retaining front portion 51 sleeves the outward flange 303 to combine with the needle unit 3 after the plunger 4 is continuously pushed to push the flexible holder-supporting seat 24, wherein the needle body 30 is fastened by the spacer portion 212. The needle unit 3 is released from the flexible holder-supporting seat 24 when the flexible holder-supporting seat 24 does not contact with the needle unit 3. As shown in FIG. 4E, the flexible sealing member 5 stops moving due to the immovable needle body 30 and then is released from the inward flange 44 because the plunger 4 is continuously pushed to push the flexible holder-supporting seat 24 and release the flexible sealing member 5. Thus the needle unit 3 combined with the flexible sealing member 5 is automatically retracted into the plunger body 40 due to the difference between two different pressures.

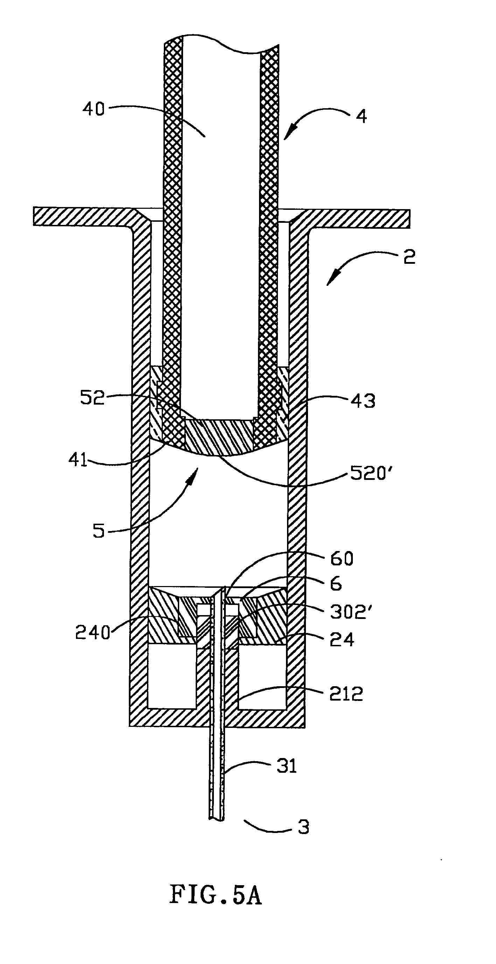

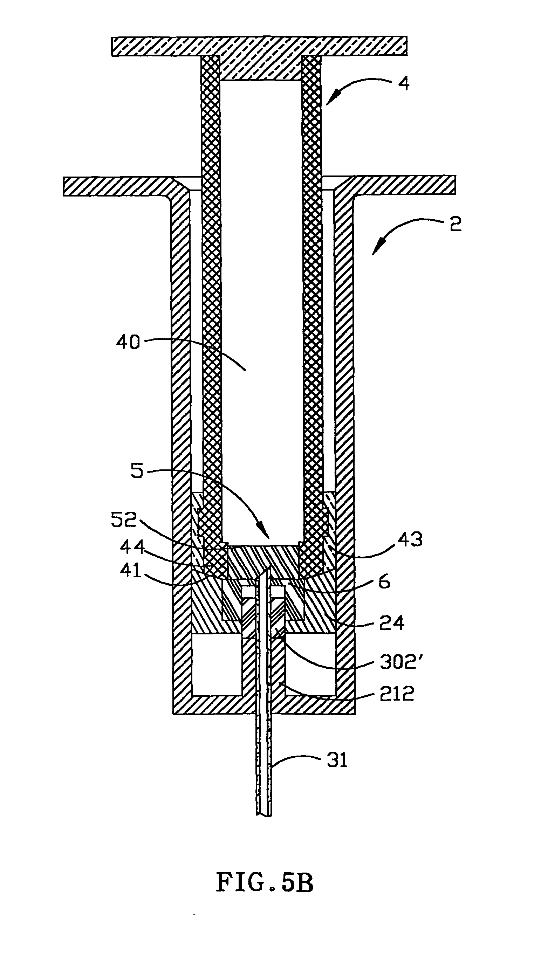

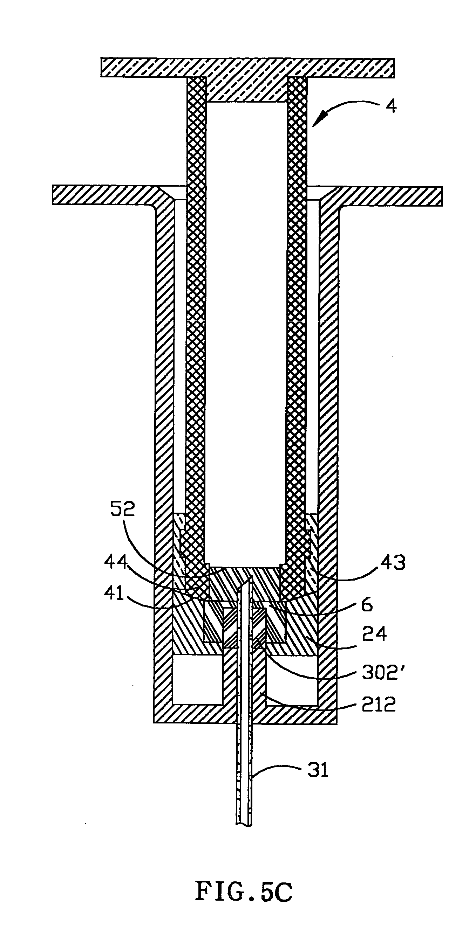

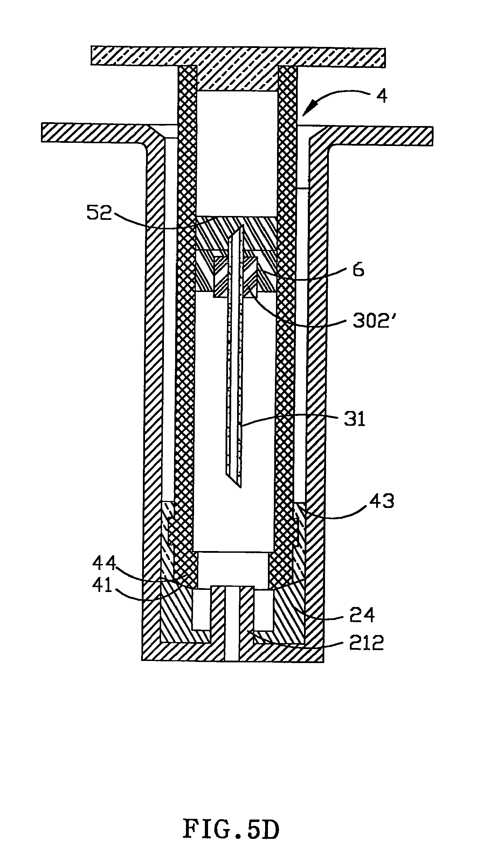

It is to be understood that many modifications and variations of the structures of the annular flexible holder-supporting seat, the sealing member and the needle unit, and the method for disposing the sealing member are possible in light of the above teachings. FIG. 5A-5D illustrates the third embodiment of the present invention. The side of the central hole 240 of the flexible holder-supporting seat 24 fastens a U-shaped flexible element 6 including a hole 60. The U-shaped flexible element 6 is clenched by the flexible holder-supporting seat 24 and the needle holder 302' . The needle 31 projected from the U-shaped flexible element 6 is clenched by the hole 60. The external diameter of the U-shaped flexible element 6 is slightly larger than the internal diameter of the plunger 4. The needle 31 is fastened by the needle holder 302' and projected from the needle holder 302' . The shape of the flexible sealing member 5 of this embodiment is also different from the shape of the flexible sealing member 5 of the above embodiments of the present invention. The curved projection 520' of the sealing rear portion 52 may be a cambered surface as shown in FIG. 5A. As shown in FIG. 5B, the needle 31 stabs into the curved projection 520' to seal the needle 31 and combine with the U-shaped flexible element 6 and the flexible sealing member 5 when the plunger 4 is pushed to contact with the flexible holder-supporting seat 24. As shown in FIG. 5C, when the plunger 4 is continuously pushed, the flexible holder-supporting seat 24 is pushed by the plunger 4 to slip and release the U-shaped flexible element 6. Because the needle holder 302' is propped up by the spacer portion 212, the U-shaped flexible element 6 is also pushed by the plunger 4 until the bottom of the U-shaped flexible element 6 contacting with the needle holder 302' . The needle 31 stabs into the flexible sealing member 5 deeper when the U-shaped flexible element 6 is pushed forward to contact with the needle holder 302' . The flexible sealing member 5 is pushed by the U-shaped flexible element 6 during the plunger 4, which then pushes the flexible holder-supporting seat 24. After the flexible holder-supporting seat 24 is pushed by the plunger 4 continuously and the inward flange 44 releasing the flexible sealing member 5, the flexible sealing member 5 combining with the needle unit 3 and U-shaped flexible element 6 is withdrawn by the lower pressure inside the plunger 4. As shown in FIG. 5D, the needle unit 3, the U-shaped flexible element 6 and the flexible sealing member 5 is withdrawn into the plunger body 40 due to the pressure.

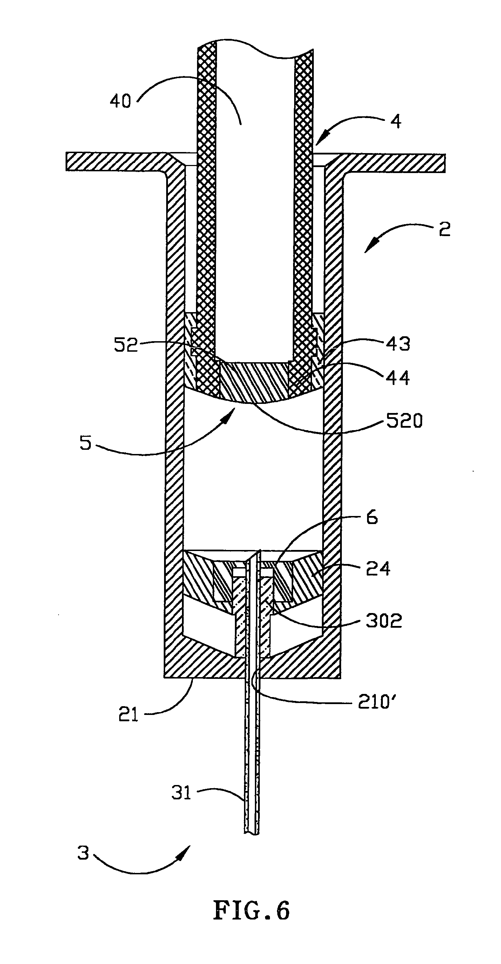

As shown in FIG. 6, the fourth embodiment of the present invention provides a safety syringe including a syringe barrel 2 with a different front-end wall 21. The front-end wall 21 includes a front opening 210' for fastening a needle 31, wherein the needle 31 projects from the front end wall 21. The needle 31 stabs into the sealing rear portion 520 to seal the needle 31 and combine the needle unit 3 with the U-shaped flexible element 6 and the flexible sealing member 5 when the plunger 4 is pushed to contact with the flexible holder-supporting seat 24. When the plunger 4 is continuously pushed, the flexible holder-supporting seat 24 is pushed by the plunger 4 to slip and release the U-shaped flexible element 6. Because the 210 prop up the needle holder 302, the U-shaped flexible element 6 is also pushed by the plunger 4 until the bottom of the U-shaped flexible element 6 contacting with the needle holder 302. The needle 31 stabs into the flexible-sealing member 5 deeper when the U-shaped flexible element 6 is pushed to contact with the needle holder 302. The flexible sealing member 5 is pushed by the U-shaped flexible element 6 during the plunger 4 pushes the flexible holder-supporting seat 24. After the flexible holder-supporting seat 24 being continuously pushed by the plunger 4 and the inward flange 44 releasing the flexible sealing member 5, the flexible sealing member 5 combining with the needle unit 3 and U-shaped flexible element 6 is withdrawn by the lower pressure inside the plunger 4. The needle unit 3, the U-shaped flexible element 6 and the flexible-sealing member 5 is withdrawn into the plunger body 40 due to the pressure.

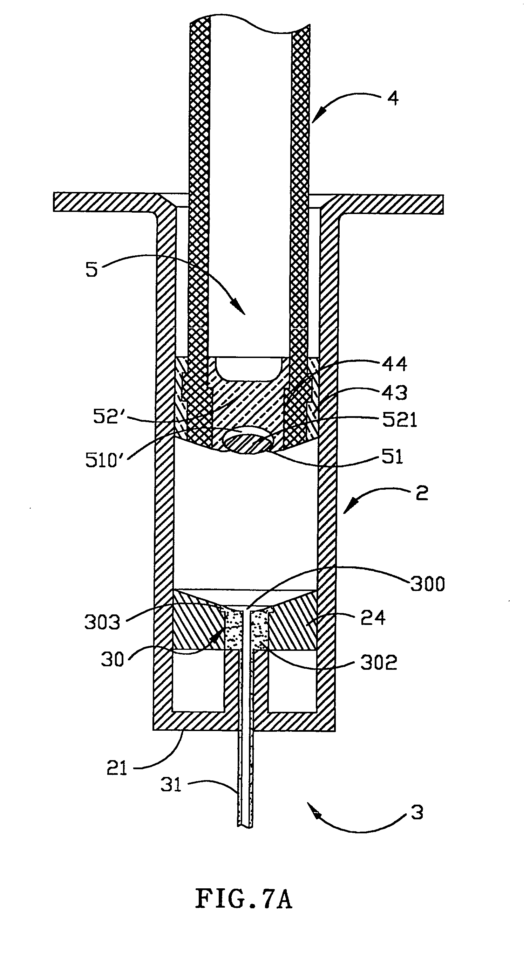

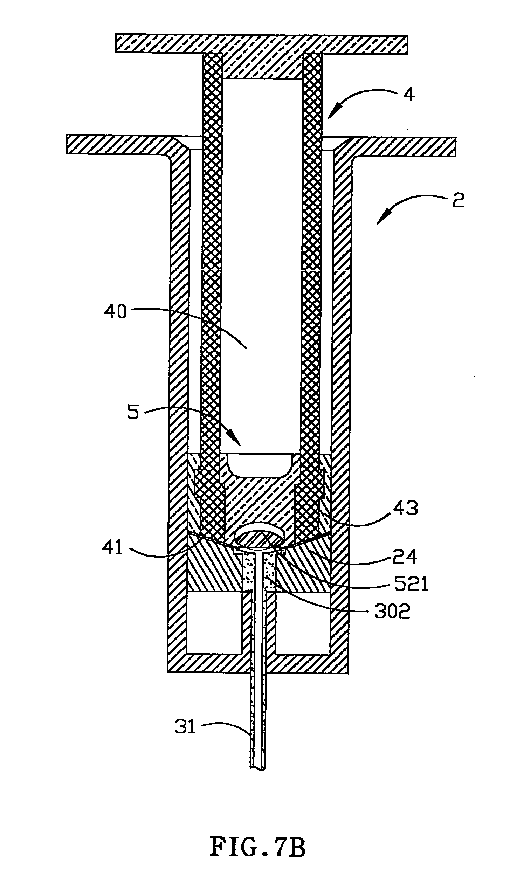

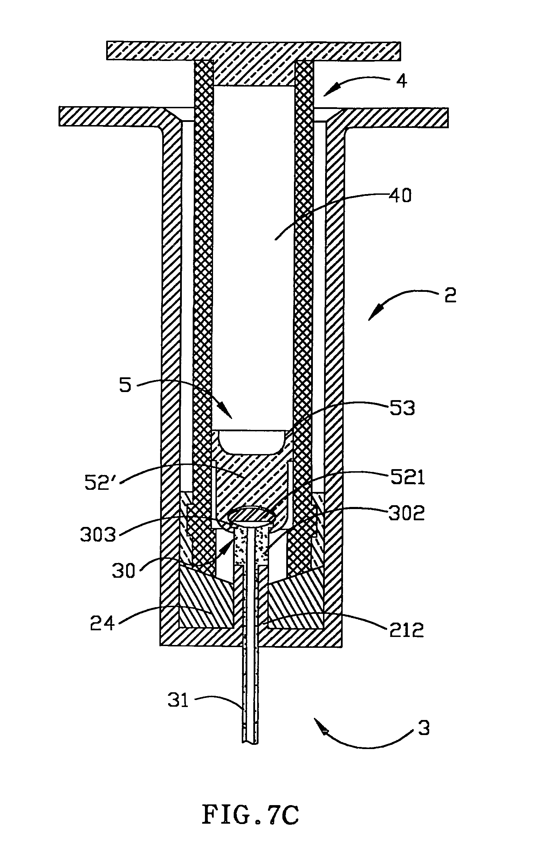

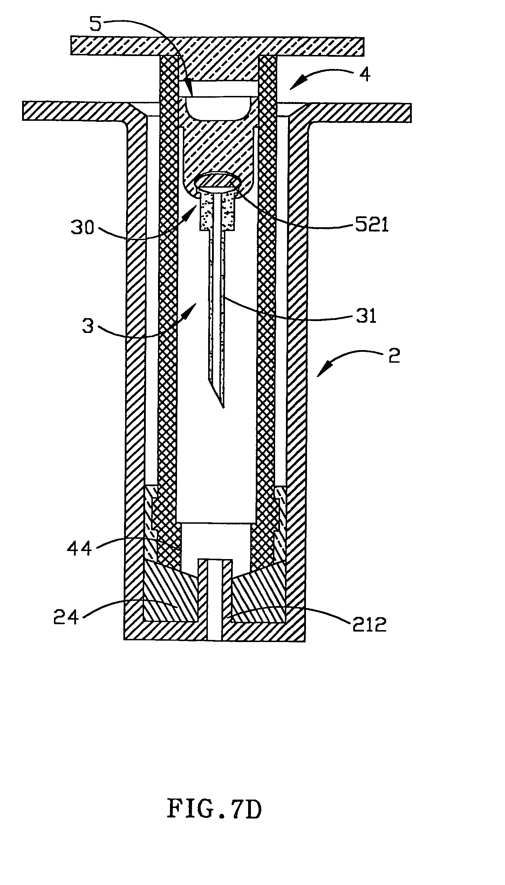

The fifth embodiment of the present invention is illustrated as FIG. 7A-7D. The difference between the first embodiment and the fifth embodiment of the present invention is the structure of the flexible sealing member 5. As shown in FIG. 7A, the flexible sealing member 5 includes a holder-retaining front portion 51, a blind hole 510' and a piston body 521, wherein the blind hole 510' is formed within the holder-retaining front portion 51. As shown in FIG. 7B, the holder-retaining front portion 51 becomes slightly flat, because the holder-retaining front portion 51 contacts with the needle body 30 when the plunger 4 is pushed forward to be close to the flexible holder-supporting seat 24. As shown in FIG. 7C, the slightly flat holder-retaining front portion 51 is continuously pushed to combine with the outward flange 303 when the plunger 4 is continuously pushed. When the inward flange 44 is still pushed forward to push the flexible holder-supporting seat 24, the piston body 521 contacts with the rear opening 300 to stuff the rear opening 300 to lock the needle 31 from injecting the medicament. The holder-retaining front portion 51 sleeves the outward flange 303 to combine with the needle unit 3 after the plunger 4 is pushed continuously to push the flexible holder-supporting seat 24, wherein the needle body 30 is propped by the spacer portion 212. As shown in FIG. 7D, the needle unit 3 is released from the flexible holder-supporting seat 24 when the flexible holder-supporting seat 24 does not contact with the needle unit 3. The flexible sealing member 5 stops moving due to the immovable needle body 30 and then is released from the inward flange 44 because the plunger 4 is continuously pushed to push the flexible holder-supporting seat 24 and release the flexible sealing member 5. Thus the needle unit 3 combined with the flexible sealing member 5 and the piston body 521 is automatically retracted into the plunger body 40 due to the different pressures.

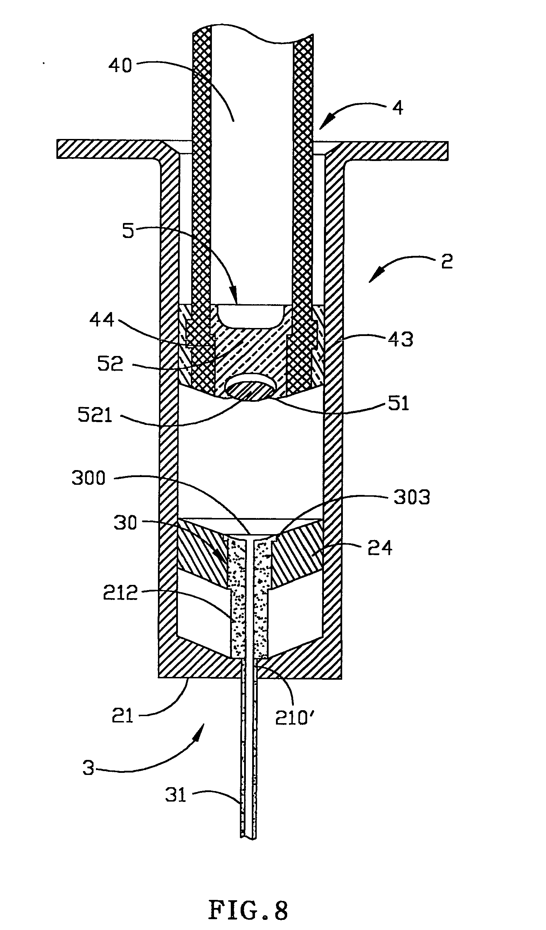

As shown in FIG. 8, the sixth embodiment of the present invention provides a safety syringe including a syringe barrel 2 with a different front-end wall 21. The front-end wall 21 includes a front opening 210' for fastening a needle 31, wherein the needle 31 projects from the front end wall 21. The holder-retaining front portion 51 is pushed to combine with the outward flange 303 when the plunger 4 is pushed to be close to the flexible holder-supporting seat 24. When the inward flange 44 is still pushed forward to push the flexible holder-supporting seat 24, the piston body 521 contacts with the rear opening 300 to stuff the rear opening 300 to lock the needle 31 from injecting the medicament. The holder-retaining front portion 51 sleeves the outward flange 303 to combine with the needle unit 3 after the plunger 4 is continuously pushed to push the flexible holder-supporting seat 24, wherein the needle body 30 is propped by the spacer portion 212. The needle unit 3 is released from the flexible holder-supporting seat 24 when the flexible holder-supporting seat 24 does not contact with the needle unit 3. The flexible sealing member 5 stops moving due to the immovable needle body 30 and the piston body 521, and then the flexible sealing member 5 is released from the inward flange 44 because the plunger 4 is continuously pushed to push the flexible holder-supporting seat 24. Thus the needle unit 3 combined with the flexible sealing member 5 and the piston body 521 is automatically retracted into the plunger body 40 due to the difference between two different pressures.

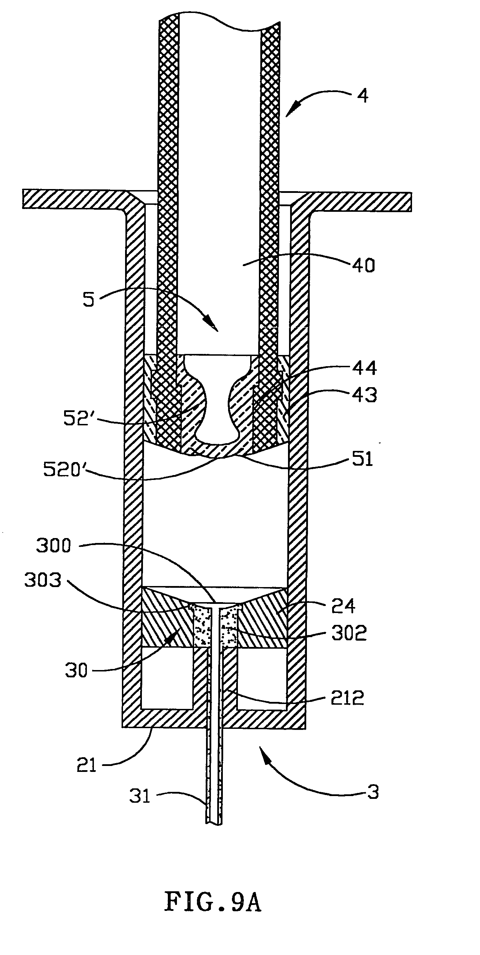

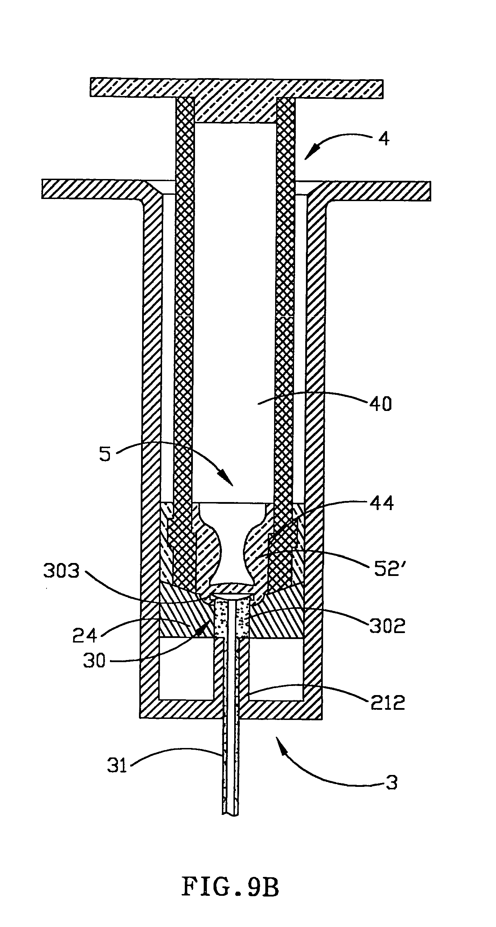

The seventh embodiment of the present invention is illustrated as FIG. 9A and FIG. 9B. The difference between the fifth embodiment and the seventh embodiment of the present invention is the structure of the flexible sealing member 5. As shown in FIG. 9A, the flexible sealing member 5 includes a holder-retaining front portion 51, a sealing rear portion 52' and a curved projection 520', wherein the holder-retaining front portion 51 is a hollow awl extended forward from the hollow sealing rear portion 52'. The sealing rear portion 52' includes a hollow space therein. The external diameter of the holder-retaining front portion 51 is as small as that extending forward. The surface of the curved projection 520' is a cambered surface formed on the hollow sealing rear portion 52'. As shown in FIG. 9B, the holder-retaining front portion 51 is pushed to combine with the outward flange 303 when the plunger 4 is pushed forward to be close to the flexible holder-supporting seat 24 when the plunger 4 is pushed continuously. When the inward flange 44 is still pushed forward to push the flexible holder-supporting seat 24, the curved projection 520' contacts with the rear opening 300 to stuff the rear opening 300 to prevent the needle 31 from injecting the medicament. The holder-retaining front portion 51 sleeves the outward flange 303 to combine with the needle unit 3 after the plunger 4 is pushed continuously to push the flexible holder-supporting seat 24, wherein the needle body 30 is propped by the spacer portion 212. The needle body 30 is released from the flexible holder-supporting seat 24 when the flexible holder-supporting seat 24 does not contact with the needle unit 3. The flexible sealing member 5 stops moving due to the immovable needle body 30 and is then released from the inward flange 44 because the plunger 4 is continuously pushed to push the flexible holder-supporting seat 24 and release the flexible sealing member 5. Thus the needle unit 3 combined with the flexible sealing member 5 is automatically retracted into the plunger body 40 due to the difference between two different pressures.

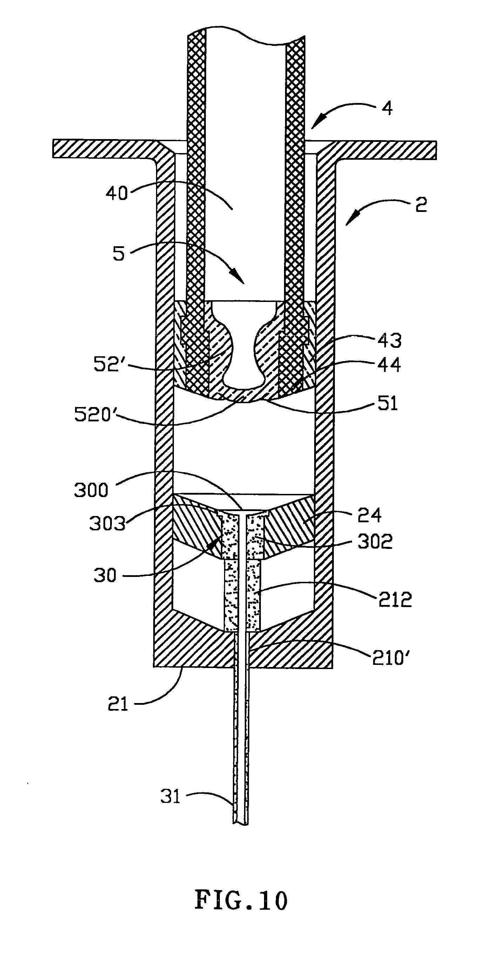

As shown in FIG. 10, the eighth embodiment of the present invention provides a safety syringe including a syringe barrel 2 with a different front-end wall 21. The front-end wall 21 includes a front opening 210' for fastening a needle 31, wherein the needle 31 projects from the front end wall 21. The holder-retaining front portion 51 is pushed to sleeve and combine with the outward flange 303 when the plunger 4 is pushed to be close to the flexible holder-supporting seat 24. When the inward flange 44 is still pushed forward to push the flexible holder-supporting seat 24, the curved projection 520' contacts with the rear opening 300 to stuff the rear opening 300 to prevent the needle 31 from injecting the medicament. The holder-retaining front portion 51 sleeves the outward flange 303 to combine with the needle unit 3 after the plunger 4 is continuously pushed to push the flexible holder-supporting seat 24, wherein the needle holder 302 is propped by the spacer portion 212. The needle unit 3 is released from the flexible holder-supporting seat 24 when the flexible holder-supporting seat 24 does not contact with the needle unit 3. The flexible sealing member 5 stops moving due to the immovable needle body 30, and then the flexible sealing member 5 is released from the inward flange 44 because the plunger 4 is continuously pushed to push the flexible holder-supporting seat 24. Thus the needle unit 3 combined with the flexible sealing member 5 is automatically retracted into the plunger body 40 due to the difference between two different pressures.

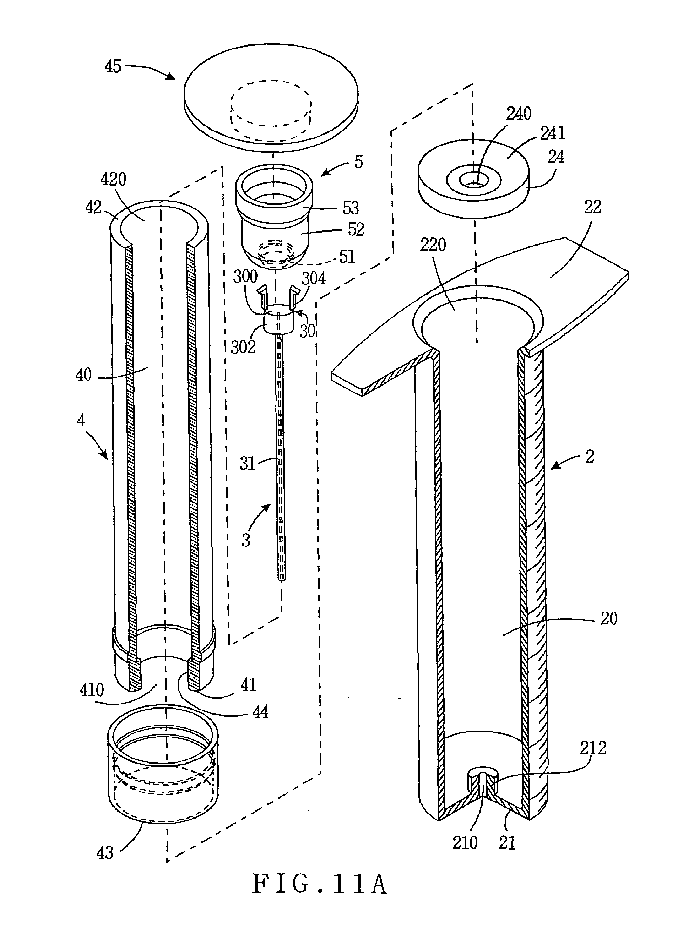

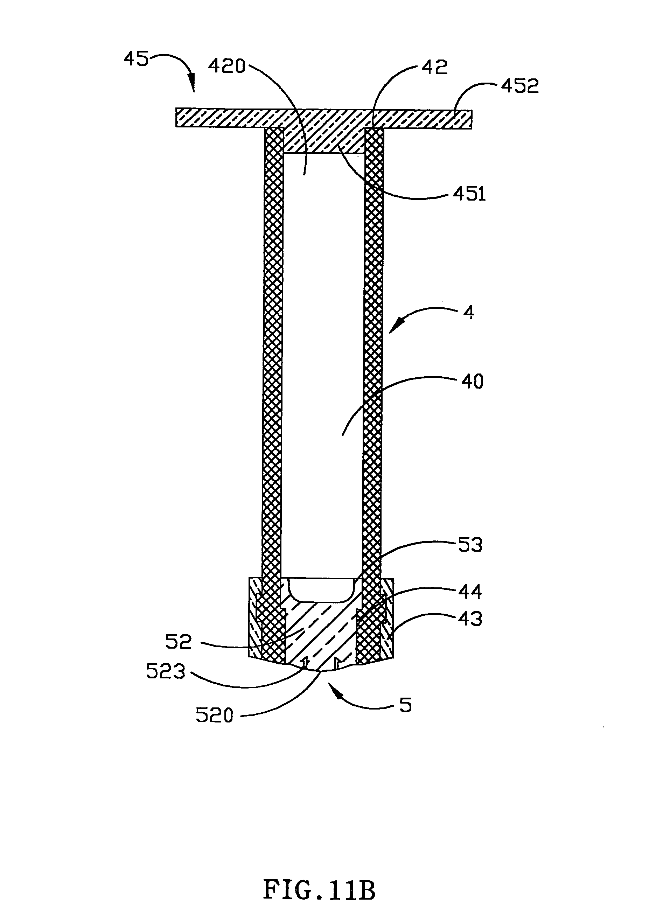

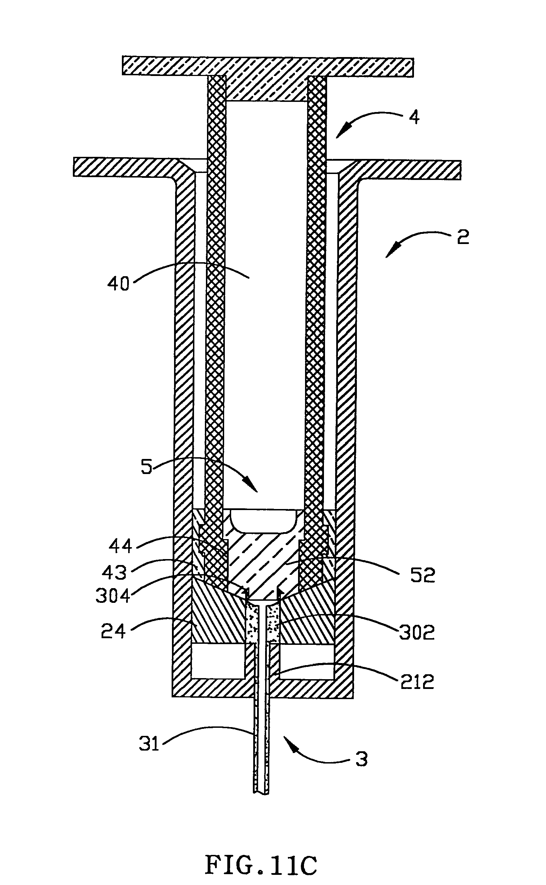

The ninth embodiment of the present invention is illustrated as FIG. 11A to FIG. 11C. The difference between the ninth embodiment and the first embodiment of the present invention is the structure of the needle unit 3 and the flexible-sealing member 5. As shown in FIG. 11A, that the needle unit 3 includes a needle holder 302 and a crook 304, wherein the crook 304 is fastened on the needle holder 302. As shown in FIG. 11B, the flexible sealing member 5 includes a sealing rear portion 52 and a curved projection 520, wherein the curved projection 520 is a cambered surface. An opening 523 is formed inside the sealing rear portion 52 for wedging the crook 304 of the needle body 30. As shown in FIG. 11C, the opening 523 of the sealing rear portion 52 is pushed to wedge the crook 304 for combining with the needle body 30 when the plunger 4 is pushed forward to be close to the flexible holder-supporting seat 24. When the open front end 41 of the plunger 4 is pushed to be close to the flexible holder-supporting seat 24, the curved projection 520 contacts with the rear opening 300 to stuff the rear opening 300 to lock the needle 31 from injecting the medicament. The flexible sealing member 5 stops moving due to the immovable needle body 30 propped by the spacer portion 212, after the plunger 4 is continuously pushed to push the flexible holder-supporting seat 24. The needle body 30 is released from the flexible holder-supporting seat 24 when the flexible holder-supporting seat 24 does not contact with the needle unit 3. The flexible sealing member 5 stops moving due to the immovable needle body 30 and then is released from the inward flange 44 because the plunger 4 is continuously pushed to push the flexible holder-supporting seat 24 and release the flexible sealing member 5. Thus the needle unit 3 combined with the flexible sealing member 5 is automatically retracted into the plunger body 40 due to the difference between two different pressures.

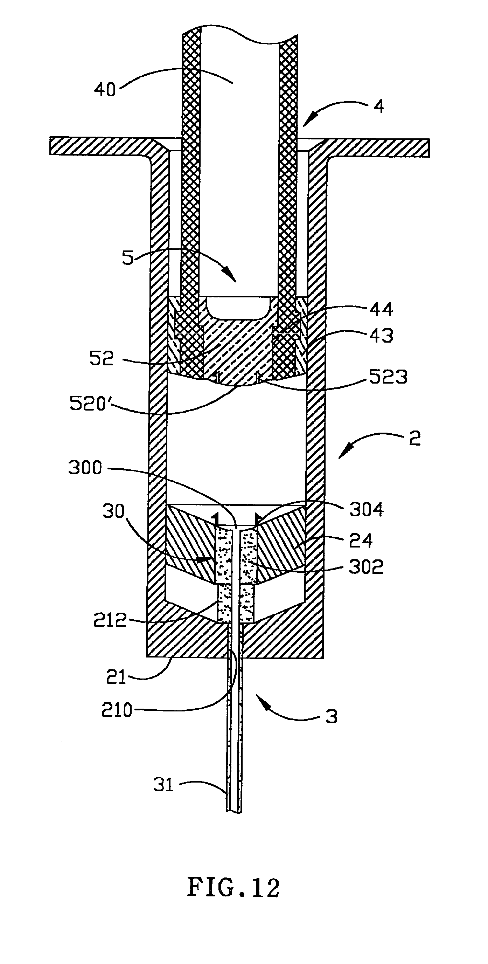

As shown in FIG. 12, the tenth embodiment of the present invention provides a safety syringe including a syringe barrel 2 with a different front-end wall 21. The front-end wall 21 includes a front opening 210 for fastening a needle 31, wherein the needle 31 projects from the front-end wall 21. The curved projection 520 is pushed to wedge the crook 304 and combine with the needle body 30 when the plunger 4 is pushed to be close to the flexible holder-supporting seat 24. When the inward flange 44 is still pushed forward to push the flexible holder-supporting seat 24, the curved projection 520 contacts with the rear opening 300 to stuff the rear opening 300 to prevent the needle 31 from injecting the medicament. The flexible sealing member 5 stops moving due to the immovable needle body 30 propped by the spacer portion 212, after the plunger 4 is continuously pushed to push the flexible holder-supporting seat 24. The needle body 30 is released from the flexible holder-supporting seat 24 when the flexible holder-supporting seat 24 does not make contact with the needle body 30. The flexible sealing member 5 stops moving due to the immovable needle body 30, and then the flexible sealing member 5 is released from the inward flange 44 because the plunger 4 is continuously pushed to push the flexible holder-supporting seat 24. Thus the needle unit 3 combined with the flexible sealing member 5 is automatically retracted into the plunger body 40 due to the difference between two different pressures.

The present invention provides a safety syringe including simple elements. The present invention also economizes the cost for manufacturing the safety syringes and effectively protects the operating staff from becoming stabbed.

Obviously, many modifications and variations of the present invention are possible in light of the above teachings. It is to be understood that within the scope of the appended claims, the present invention may be practiced other than as specifically described herein.

Although the specific embodiments have been illustrated and described, it will be obvious to those skilled in the art that various modifications may be made without departing from what is intended to be limited solely by the appended claims.

## Claims

1. A safety syringe having a syringe barrel (2), a needle unit (3), a plunger (4), and a flexible scaling member (5), wherein said needle unit (3) is set in front end of the syringe barrel (2), said plunger (4) movably disposed within said syringe barrel (20), and said flexible sealing member (5) disposed movably within said front end of said plunger (4),
said syringe barrel (2) has a barrel body (20) with a front opening (210), a rear opening (220), a front-end wall (21) and a rear end wall (22), wherein a cross-sectional area of said front opening (210) is smaller than a cross-sectional area of said rear opening (220), a spacer portion (212) is penetrated by said front opening (210);
a flexible holder-supporting seat (24) being disposed movably within said syringe barrel (2) and on said spacer portion (212), wherein said flexible holder-supporting seat (24) includes a central hole (240) formed there through;
said needle unit (3) has a needle holder (302) and a needle (31) penetrating said needle holder (302), said needle unit (3) is disposed movably within said front end wall, said needle holder (302) being sleeved by said central hole of said flexible holder-supporting seat (24) on said spacer portion (212), and a U-shaped flexible element (6) being fastened by said flexible holder-supporting seat (24) and said needle holder (302), wherein a rear opening (300) is formed in said rear end of said needle holder (302), said needle (31) penetrates said needle holder (302) and said spacer portion (212) and is projected from said front opening;
said plunger (4) has a front end with a front opening (300) and an inward flange(44), a rear end and a plunger body (40) formed between said front end and said rear end (42), a cross-sectional area of front opening being larger than an external diameter of said needle holder (302), said plunger (4) being disposed movably within said syringe barrel (2), and a rubber seal ring (43) sleeved around said plunger (4), wherein an external diameter of said rubber scaling ring (43) is larger than said internal diameter of said syringe barrel (2); and
said flexible sealing member (5) sleeved by said inward flange(44) to seal said front opening of said plunger (4), said flexible scaling member (5) combined with said needle unit (3) after said plunger(4) being pushed to be close to said flexible holder-supporting seat (24) due to said plunger (4), wherein said flexible sealing member (5) is propped by said needle holder (302), said flexible holder-supporting seat (24) is sleeved on said spacer portion (212) and then said flexible scaling member (5) is released from said inward flange (44) to be automatically retracted into said plunger body (40) due to an air pressure which is sealed in said plunger (4), **characterized in that** said space portion is disposed integrally within said barrel body (20).

2. The safety syringe according to claim 1, wherein said needle holder (302) includes an outward flange (303), an external diameter of said outward flange (303) is larger than a diameter of said needle holder (302).

3. The safety syringe according to claim 1, wherein said flexible sealing member (5) includes a sealing rear portion (52) and a holder-retaining front portion (51), said an external diameter of said sealing rear portion (52) is larger than an internal diameter of said inward flange (44), said holder-retaining front portion (51) is extended from said scaling rear portion (52) and includes a blind hole (510), an internal diameter of said blind hole (510) is smaller than said external diameter of said outward flange (303).

4. The safety syringe according to claim 3, wherein said flexible sealing member (5) includes a curved projection (520').

5. The safety syringe according to claim 3, further comprising a piston body (521) disposed within said blind hole (510).

6. The safety syringe according to claim 3, wherein said sealing rear portion (52) includes a hollow spacer.

7. The safety syringe according to claim 1, wherein said needle unit (3) includes a crook (304).

8. The safety syringe according to claim 1, wherein said flexible sealing member (5) includes a sealing rear portion (52) and an opening formed within said sealing rear portion (52).

9. The safety syringe according to claim 1, wherein said U-shaped flexible element (6) includes a hole penetrated by said needle (31).

10. The safety syringe according to claim 1, wherein said flexible sealing member (5) includes a sealing rear portion (52).

## Patentansprüche

1. Eine Sicherheitsspritze, die einen Spritzenhohlraum (2) aufweist, eine Nadeleinheit (3), einen Kolben (4) und ein flexibles Abdichtelement (5), wobei besagte Nadeleinheit (3) am vorderen Ende des Spritzenhohlraums (2) angesetzt ist und der besagte Kolben (4) ist beweglich innerhalb des Spritzenhohlraums (2) angeordnet und das besagte flexible Abdichtungselement (5) ist beweglich innerhalb des vorderen Endes des besagten Kolbens (4) angeordnet,
der besagte Spritzenhohlraum (2), hat einen Hohlraumkörper (20) mit einer vorderen Öffnung (210), einer hinteren Öffnung (220), einer vorderen Endwand (21) und eine hinteren Endwand (22), wobei eine Querschnittsfläche der besagten vorderen Öffnung (210) kleiner als eine Querschnittsfläche der besagten hinteren Öffnung (220) ist, ein Beabstandungsbereich (212) wird durch die besagte vorderer Öffnung (210) penetriert;
ein flexibler Halterunterstützungssitz (24) ist beweglich innerhalb des besagten Spritzenhohlraums (2) und auf dem besagten Beabstandungsbereieh (212) angeordnet, wobei der besagte flexible Halterunterstützungssitz (24) ein zentrales Loch (240) umfasst, das durchbrechend ausgebildet ist;
besagte Nadeleinheit (3) weist einen Nadelhalter (302) auf und eine Nadel (31), die diesen Nadelhalter penetriert (302), die besagte Nadeleinheit (3) ist beweglich innerhalb der vorderen Endwand angeordnet, der besagte Nadelhalter (302) wird umhüllt durch das besagte zentrale Loch des flexiblen Halterunterstützungssitzes (24) auf dem besagten Beabstandungsbereich (212), und ein U-förmig flexibles Element (6), das befestigt wurde durch den besagten flexiblen Halteunterstützungssitz (24) und dem besagten Nadelhalter (302), wobei eine hintere Öffnung (300) im hinteren Ende des besagten Nadelhalters (302) ausgebildet ist, die besagte Nadel (31) penetriert den besagten Nadelhalter (302) und besagter Beabstandungsbereich (212) ist projektiert von der besagten Öffnung;
besagter Kolben (4) weist ein vorderes Ende auf mit einer vorderen Öffnung (300) und einen nach innen gerichteten Flansch (44), ein hinteres Ende und einen Kolbenkörper (40), der zwischen dem besagten vorderen Ende und dem besagten hinteren Ende (42) ausgebildet ist, eine Querschnitfläche der vorderen Öffnung ist größer als der externe Durchmesser des besagten Nadelhalters (302), der besagte Kolben (4) ist beweglich innerhalb des besagten Spritzenhohlraums (2) angeordnet und ein Gummidichtungsring (43) umhüllten den besagten Kolben (4), wobei ein externer Durchmesser des besagten Gummidichtungsrings (43) größer ist als der interne Durchmesser des besagten Spritzenhohlraums (2); und
ein flexibles Abdichtelement (5) ist umhüllt durch den besagten nach innen gerichteten Flansch (44), um die besagte vordere Öffnung des besagten Kolbens (4) abzudichten, das besagte flexible Abdichtelement (5) wird mit der besagten Nadeleinheit (3) kombiniert, nachdem der besagte Kolben (4) geschoben wird, um aufgrund des besagten Kolbens (4), nahe zu der besagten flexiblen Halterunterstützungssitz (24) zu sein, das besagte flexible Abdichtelement (5) wird durch den besagten Nadelhalter (302) gestützt, der besagte flexible Halterunterstützungssitz (24) wird auf den besagten Beabstandungsbereich (212) gehüllt und dann wird das flexible Abdichtelement (5) losgelassen von dem nach innen gerichteten Flansch (44), um automatisch in den besagten Kolbenkörper (40) hereingezogen zu werden, aufgrund eines Luftdrucks, der in dem besagten Kolben (4) abgedichtet ist, **dadurch gekennzeichnet, dass** der besagte Beabstandungsbereich integral mit dem besagten Hohlraumkörper (20) ausgebildet ist.

2. Die Sicherheitsspritze gemäß Anspruch 1, wobei der besagte Nadelhalter (302) einen nach außen gerichteten Flansch (303) umfasst, einen äußeren Durchmesser des besagten nach außen gerichteten Flansches (303), der größer ist als der Durchmesser des besagten Nadelhalters (302).

3. Die Sicherheitsspritze gemäß Anspruch 1, wobei das flexible Abdichtelement (5) einen hinteren Abdichtbereich (52) aufweist und einen Halter-rückhaltenden Frontbereich (51), der besagte äußere Durchmesser des besagten hinteren Abdichtbereichs (52) ist größer als der innere Durchmesser des besagten nach innen gerichteten Flansches (44), der besagte Halter-rückhaltende-Frontbereich (21) erstreckt sich von den besagten abdichtenden hinteren Bereich (52) und umfasst eine Blindbohrung (510), ein innerer Durchmesser, der besagten Blindbohrung (510) ist kleiner als der äußere Durchmesser des besagten nach außen gerichteten Flansches (303).

4. Die Sicherheitsspritze gemäß Anspruch 3, wobei das besagte flexible Abdichtelement (5) einen abgerundeter Überstand bzw. eine Projektion (520') aufweist.

5. Die Sicherheitsspritze gemäß Anspruch 3, weiterhin umfassend einen Kolbenkörper (522), der innerhalb der besagten Blindbohrung (510) angeordnet ist.

6. Die Sicherheitsspritze gemäß Anspruch 3, wobei der besagte hintere Abdichtungsbereich (52) einen hohlen Beabstander aufweist.

7. Die Sicherheitsspritze nach Anspruch 1, wobei die Nadeleinheit (3) einen Haken (304) umfasst,

8. Die Sicherheitsspritze gemäß Anspruch 1, wobei das flexible Abdichtungselement (5) einen hinteren Abdichtungsbereich (52) und eine Öffnung aufweist, die innerhalb des hinteren Abdichtungsbereichs (52) ausgebildet ist.

9. Die Sicherheitsspritze gemäß Anspruch 1, wobei das besagte U-förmige flexible Element (6) ein Loch enthält, das durch die besagte Nadel (31) penetriert wird.

10. Die Sicherheitsspritze gemäß Anspruch 1, wobei das besagte flexible Abdichtungselement (5) einen abdichtenden hinteren Bereich (52) aufweist.

## Revendications

1. Seringue de sécurité ayant un cylindre de seringue (2), une unité d'aiguille (3), un piston (4), et un élément de graduation flexible (5), dans laquelle ladite unité d'aiguille (3) est fixée à une extrémité avant du cylindre de seringue (2), ledit piston (4) étant disposé de manière mobile dans ledit cylindre de seringue (20), et ledit élément d'étanchéité flexible (5) étant disposé de manière mobile dans ladite extrémité avant dudit piston (4),
ledit cylindre de seringue (2) a un corps de cylindre (20) doté d'une ouverture avant (210), une ouverture arrière (220), une paroi d'extrémité avant (21) et une paroi d'extrémité arrière (22), dans lequel une superficie transversale de ladite ouverture avant (210) est inférieure à une superficie transversale de ladite ouverture arrière (220), ladite ouverture avant (210) pénétrant dans une pièce d'espacement (212) ;
un logement supportant un support flexible (24) disposé de manière mobile dans ledit cylindre de seringue (2) et sur ladite pièce d'espacement (212), dans lequel ledit logement supportant le support flexible (24) comprend un orifice central (240) ménagé à travers lui ;
ladite unité d'aiguille (3) est dotée d'un support d'aiguille (302) et d'une aiguille (31) pénétrant dans ledit support d'aiguille (302), ladite unité d'aiguille (3) étant disposée de manière mobile dans ladite paroi d'extrémité avant, ledit support d'aiguille (302) étant gainé par ledit orifice central dudit logement supportant de support flexible (24) sur ladite partie d'espacement (212), et un élément flexible en forme de U (6) étant fixé par ledit logement supportant le support flexible (24) et ledit support d'aiguille (302), dans lequel une ouverture arrière (300) est ménagée dans ladite extrémité arrière dudit support d'aiguille (302), ladite aiguille (31) pénétrant dans ledit support d'aiguille (302) et dans ladite partie d'espacement (212) et étant projetée depuis ladite ouverture avant ;
ledit piston (4) a une extrémité avant avec une ouverture avant (300) et une bride vers l'intérieur (44), une extrémité arrière et un corps de piston (40) formé entre ladite extrémité avant et ladite extrémité arrière (42), une superficie transversale de l'ouverture avant étant supérieure à un diamètre extérieur dudit support d'aiguille (302), ledit piston (4) étant disposé de manière mobile dans ledit cylindre de seringue (2) et un joint de caoutchouc (43) gainé autour dudit piston (4), dans lequel un diamètre extérieur dudit joint de caoutchouc (43) est supérieur audit diamètre intérieur dudit cylindre de seringue (2) ; et
ledit élément d'étanchéité flexible (5) est gainé par ladite bride vers l'intérieur (44) pour sceller ladite ouverture avant dudit piston (4), ledit élément d'étanchéité flexible (5) est combiné à ladite unité d'aiguille (3) après que ledit piston (4) a été poussé de façon à s'approcher dudit logement supportant le support flexible (24) par ledit piston (4), où ledit élément d'étanchéité flexible (5) est étayé par ledit support d'aiguille (302), ledit logement supportant le support flexible (24) étant gainé sur ladite pièce d'espacement (212) et ensuite ledit élément d'étanchéité flexible (5) est libéré de ladite bride vers l'intérieur (44) de façon à être automatiquement rétracté dans ledit corps de piston (40) du fait de la pression d'air qui est scellée dans ledit piston (4), **caractérisé en ce que** ladite partie d'espacement est disposée intégralement dans ledit corps de cylindre (20).

2. Seringue de sécurité selon la revendication 1, dans laquelle ledit support d'aiguille (302) comprend une bride vers l'extérieur (303), un diamètre extérieur de ladite bride vers l'extérieur (303) étant supérieur à un diamètre dudit support d'aiguille (302).

3. Seringue de sécurité selon la revendication 1, dans laquelle ledit élément d'étanchéité flexible (5) comprend une partie arrière d'étanchéité (52) et une partie avant de retenue de support (51), ledit diamètre extérieur de ladite partie arrière d'étanchéité (52) étant supérieur au diamètre intérieur de ladite bride vers l'intérieur (44), ladite partie avant de retenue de support (51) s'étend depuis ladite partie arrière de graduation (52) et comprend un orifice borgne (510), un diamètre intérieur dudit orifice borgne (510) étant inférieur audit diamètre extérieur de ladite bride vers l'extérieur (303).

4. Seringue de sécurité selon la revendication 3, dans laquelle ledit élément d'étanchéité flexible (5) comprend une projection incurvée (520').

5. Seringue de sécurité selon la revendication 3, comprenant en outre un corps de piston (521) disposé dans ledit orifice borgne (510).

6. Seringue de sécurité selon la revendication 3, dans laquelle ladite partie arrière d'étanchéité (52) comprend une pièce d'espacement creuse.

7. Seringue de sécurité selon la revendication 1, dans laquelle ladite unité d'aiguille (3) comprend une courbure (304).

8. Seringue de sécurité selon la revendication 1, dans laquelle ledit élément d'étanchéité flexible (5) comprend une partie arrière d'étanchéité (52) et une ouverture formée dans ladite partie arrière d'étanchéité (52).

9. Seringue de sécurité selon la revendication 1, dans laquelle ledit élément flexible en forme de U (6) comprend un orifice pénétré par ladite aiguille (31).

10. Seringue de sécurité selon la revendication 1, dans laquelle ledit élément d'étanchéité flexible (5) comprend une partie arrière d'étanchéité (52).
